# EUROPEAN PATENT APPLICATION

(11) **EP 4 066 828 A1**
(43) Date of publication of application: **05.10.2022**
(21) Application number: 22164043.6
(22) Date of filing: 24.03.2022
(51) Int. Cl.: A61K 31/353, C07D 311/62, A61P 1/16, A61P 25/00, A61P 35/00

(54) **POLYMERIC PROATHOCYANIDIN COMPOSITION AND APPLICATION THEREOF**

(30) Priority: 31.03.2021 WO PCT/CN2021/084584
(71) Applicant: BELX BIO-PHARMACEUTICAL (TAIWAN) CORPORATION, TAIPEI CITY 11490 (TW)
(72) Inventor: CHANG, SHAU-FENG, 302054 Zhubei City (TW); CHUNG, I-HSIN, 813023 KAOHSIUNG CITY (TW)
(74) Representative: Goddar, Heinz J.

(57) **Abstract**

The present invention relates to a pharmaceutical composition, which is characterized in that the composition comprises an effective amount of proanthocyanidin, the monomer units of the proanthocyanidin have the chemical formula of formula (I), and a pharmaceutically acceptable carrier or salt; wherein the proanthocyanidin is a polymeric proathocyanidin with a degree of polymerization ranging from 50-65. It has good antioxidant stress and can be used to treat or prevent brain diseases and aging-related diseases caused by excessive production of ROS, or to prevent and treat aging. It can also be used for the treatment and prevention of liver disease, tumor, sarcopenia and other diseases.

## Description

### Technical field

The present invention relates to the field of anti-oxidation, in particular to a composition of polymeric proathocyanidin, and its application in the field of treating diseases related to antioxidative stress.

### Background of the Invention

Proanthocyanidins, also known as proanthocyanidins, the English name is Oligomeric Proanthocyanidins, abbreviated as OPCs or OPC. It is a polyphenolic compound that can produce anthocyanin under thermal acid treatment, which is currently internationally recognized as an effective natural antioxidant for scavenging free radicals in the human body with very strong in vivo activity. OPC is generally a reddish-brown powder, with a slight smell and astringent taste, and is soluble in water and most organic solvents. Proanthocyanidins are polyphenols condensed from monomers such as catechin ((+)-catechin), epicatechin ((-)-epicatechin) or epicatechin gallate ((-)-epicatechin-3-gallate) through C4→C8 or C4→C6 bonds. Different types of polymers are formed, according to the number of condensations and the position of the connection, such as dimers, trimers, tetramers ... decamers, etc.; specifically, according to the degree of polymerization, proanthocyanidins can be classified as oligomeric proanthocyanidins (oligomeric proathocyanidin, OPC) and polymeric proanthocyanidins (polymeric proathocyanidin, PPC). At the same time, studies have shown that with the increase of the degree of polymerization of proanthocyanidins, its solubility decreases accordingly, and the PPC is difficult to be absorbed in the human intestinal tract. Therefore, oligomeric proanthocyanidins have stronger physiological activity. Proanthocyanidins can scavenge excess free radicals in the body and improve the body's immunity. They can be used as the main active components of anticancer, anti-mutation, and cardiovascular disease drugs and as safe and non-toxic natural antioxidants, etc. They are widely used in medicine, health care, food, daily chemical and other fields.

Reactive oxygen species (ROS) are generated by endogenous and exogenous processes, and their negative effects can be counteracted by antioxidant defenses. Oxidative stress refers to a state of imbalance between oxidation and anti-oxidation in the body, which is more prone to oxidation, leading to inflammatory infiltration of neutrophils, increased secretion of proteases, and the production of a large number of oxidative intermediates. Oxidative stress is a negative effect of free radicals in the body and is considered to be an important factor in aging and disease. Aging is a process characterized by the gradual loss of tissue and organ function. The oxidative stress theory of aging is based on the assumption that age-related loss of function is due to the accumulation of ROS-induced damage. Meanwhile, oxidative stress is implicated in several age-related diseases (e.g., cardiovascular disease CVDs, chronic obstructive pulmonary disease, chronic kidney disease, neurodegenerative diseases, and cancer), including sarcopenia and frailty. Given the important role of oxidative stress in the pathogenesis of many clinical diseases and aging, antioxidant treatment can actively affect the natural course of several diseases (Ilaria Liguori, et.al., Clinical Interventions in Aging 2018, 13:757-772). It has been pointed out that the chronic inflammatory state associated with aging, called inflammaging, is associated with a variety of disease states commonly observed in the elderly population. Inflammatory aging is associated with excess ROS in cells, which can lead to oxidation and damage of cellular components, increased inflammation, and activation of cell death pathways (Li Zuo, et.al., Int. J. Mol. Sci. 2019, 20:4472). Atherosclerosis is a chronic vascular inflammatory disease associated with oxidative stress and endothelial dysfunction. Various factors accelerate the process of atherosclerotic process, such as the release of inflammatory chemokines and cytokines, the production of ROS, growth factors, and the proliferation of vascular smooth muscle cells. Inflammation and immunity are key factors in the atherosclerosis development and complications (Patricia Marchio, et.al., Oxidative Medicine and Cellular Longevity Volume 2019, Article ID 8563845, 32 pages).

The balance of ROS generation and scavenging plays an important role in living cells, and has DNA damage and carcinogenic effects, which is closely related to the occurrence and development of tumors (Guo C, et.al., Oncotarget, 2017, 8:75767-75777). It is now recognized that appropriate levels of ROS are required for a variety of cellular functions, including gene expression. Due to the increase of metabolic rate, genetic mutation and relative hypoxia, the production of ROS increased in tumor cells, excess ROS is quenched by the increase in antioxidant enzymatic and non-enzymatic pathways in the same cells. The increased ROS may lead to a variety of pathological conditions, including tumor promotion and progression, as they participate in different signaling pathways and induce DNA mutations. ROS can also trigger programmed cell death (PCD). Therapeutic strategies based on mechanisms that modulate ROS levels to treat cancer are useful (Perillo, el al. Experimental & Molecular Medicine, 2020, 52:192-203). Existing studies have also demonstrated a close correlation between programmed cell necroptosis and ROS, both of which play an important role in human physiological conditions such as inflammation regulation and cancer biology. In the experiment, several small molecules were used to eliminate cancer cells by modulating ROS and programmed necrosis (Sheng-Kai Hsu, et.al., Cancers, 2020, 12:2185).

At present, the research on proanthocyanidins mainly focuses on OPC, that is, oligomeric flavonoids with a degree of polymerization of 2-4, because a large number of studies have shown that OPC have better physiological activities than monomers and polymers. For example, it is used as an additive with ultraviolet damage repairing effect in food, medicine, cosmetics, and other fields; and as an antioxidant stress of ROS, it can be used in the fields such as oxidative stress, liver function and pathology, and motor control. Although PPC are easier to obtain, they are difficult to be absorbed by the human body because of their large molecular weight, and because the steric hindrance effect will affect the activity of the phenolic hydroxyl group, biological activity is reduced and other problems are raised, which makes them be rarely studied, and even be recognized as invalid in the industry.

In the research of this application, it is unexpectedly found that PPC with a certain degree of polymerization can achieve better antioxidant stress than proanthocyanidins with a lower degree of polymerization, and have significant effects in the treatment and prevention of liver diseases, tumors, sarcopenia and other fields.

### Summary of the Invention

The object of the present invention is to provide a pharmaceutical composition, especially a pharmaceutical composition containing polymeric proanthocyanidin, which has a good ability to inhibit the production of ROS, and has significant effects in the treatment and prevention of diseases such as liver disease, influenza, sarcopenia and so on.

The invention discloses a pharmaceutical composition comprising an effective amount of proanthocyanidin, wherein the monomer units of the proanthocyanidin have the following formula: 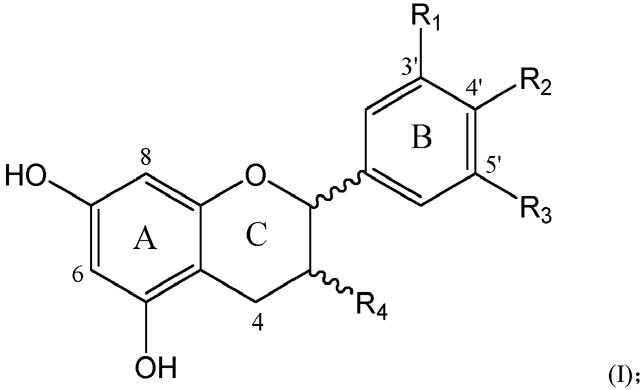 wherein, when R₁ is OCH3, R₂ is OH and R₃ is H, when R₁ is OH, R₂ is H and R₃ is H, when R₁ is OH, R₂ is OH and R₃ is H, or when R₁ is OH, R₂ is OH and R₃ is OH; and R₄ is 3-(α)-OH, 3-(β)-OH, 3-(α)-O-sugar or 3-(β)-O-sugar; wherein the proanthocyanidin has a degree of polymerization ranging from 50 to 65; and a pharmaceutically acceptable carrier or salt.

The monomer units of the proanthocyanidin are bonded to each other via C4-C8 bonding, C4-C6 bonding, or C2-O7 bonding. The monomer units of the proanthocyanidin further comprise R or S optical isomers at C2, C3 or C4.

The monomer units of the proanthocyanidin comprise flavonoid compounds such as gallocatechin, galloepicatechin or epigallocatechin.

The pharmaceutical composition disclosed in the present invention comprises catechin, epicatechin, epiafzetechin, gallates, flavonols, flavandiols, leucocyanidins or procynidins and flavan-3-ol.

The pharmaceutical composition disclosed in the present invention is extracted from the plants of *Ericaceae, Rosaceae, Pinaceae, Vitaceae* or *Urticaceae,* the plants of *Urticaceae* including *Boehmeria nivea L. Gaud.*

The proanthocyanidin of the pharmaceutical composition disclosed in the present invention has a degree of polymerization ranging from 50 to 65, preferably ranging from 55-65, more preferably 55-60.

The pharmaceutically acceptable salt of the pharmaceutical composition disclosed in the present invention includes sodium salt, potassium salt, amine salt, magnesium salt, calcium salt, zinc salt, aluminum salt, zirconium salt, dicyclohexylamine salt, methyl-D-glucosamine salt, arginine salt, lysine salt, histidine salt or glutamine salt.

The pharmaceutical composition disclosed in the present invention can be used to treat or prevent brain diseases, aging related diseases caused by the generation of active oxides, or to prevent and treat aging.

The pharmaceutical composition disclosed in the present invention can be used for treating and preventing diseases such as liver disease, tumor and sarcopenia.

### BRIEF DESCRIPTION OF THE DRAWINGS

Figure 1: Size Exclusion Chromatography (SEC) of proanthocyanidin sample 1 using multi-angle laser light scattering spectroscopy (MALS) as the detector.
Figure 2: Mass distribution analysis of proanthocyanidin sample 1 using multi-angle laser light scattering spectroscopy (MALS) as the detector.
Figure 3: The diagram of the repeatability experiment of the mass distribution analysis of proanthocyanidin sample 1.
Figure 4: Plot of different weight fractions versus elution volumes for proanthocyanidin sample 1.
Figures 5A and 5B: Analysis of specific refractive index increment of PPC polymer respectively.
Figure 6: The experimental graph of the inhibition of ROS production in hepatoma cells (Huh7) by PPC polymers.
Figure 7: The experimental graph of the inhibition of ROS production in colorectal cancer cells (HRT-18) by PPC polymers.
Figure 8: The experimental comparison of the inhibition of ROS production between PPC polymers and OPC polymers in colorectal cancer cells (HRT-18).
Figure 9: PPC polymers improve the effect of tumor-secreted factors (lung cancer conditioned medium, LLC) on inhibition of C2C12 myotubes.
Figures 10A and 10B: PPC polymers inhibit the expression of tumor-secreted factor (LLC)-induced C2C12 myotube cell atrophy-related genes Atrogin-1 and MuRF-1, respectively.
Figure 11: PPC polymers inhibit tumor-secreted factor (LLC)-induced IL-6 production by C2C12 myotubes.
Figure 12: PPC polymers ameliorate C2C12 myotube cell injury induced by MnCl₂.
Figures 13A and 13B: PPC polymers inhibit the expression of genes Atrogin-1 and MuRF-1 associated with C2C12 myotube cell injury induced by H₂O₂.
Figure 14: PPC polymers inhibit H₂O₂-induced IL-6 production by C2C12 myotubes.

### DETAILED DESCRIPTION

The technical solutions of the present invention will be described in detail below with reference to the accompanying drawings. It should be understood that the specific embodiments described herein are only used to explain the present invention, but not to limit the present invention. The scope of the present application is not limited by these embodiments, but is subject to the scope of the patent application.

In the disclosure, the proanthocyanidins may be extracted from plants. The used plant may comprise a *Leguminosae, Crassulaceae, Combretaceae, Asclepiadaceae, Rosaceae, Ericaceae, Pinaceae, Lamiaceae, Polygonaceae, Vitaceae or Urticaceae,* preferably *Boehmeria nivea L. Gaud* of the Urticaceae. The extracted part of the plant may comprise roots, stems, leaves and/or fruits.

In the disclosure, the plant may be extracted using general known methods. In one embodiment, dried roots, stems, leaves and/or fruits of a plant are sliced or grated. Next, the plant is extracted using an extraction solution. In one embodiment, roots and/or stems of the *Boehmeria nivea* L. Gaud. is selected for extract.

The extraction solution may be selected from water or a solution mixed by water and solvents with different polarities from water. The solvents with different polarities from water may comprise ethanol, acetone, methanol or ethyl acetate. The solvents may be used alone, mixed with each other, or mixed with water. The ratio of the extraction solution to the plant has no particular limitation. In one embodiment, the ratio of the extraction solution to the plant is 1:10 (W/W).

During the extraction, the extraction temperature may be slightly changed as different extraction solutions are selected. In one embodiment, the plant may be immersed in an extraction solution at room temperature. In another embodiment, the extraction solution may be heated to the reflux temperature (60-100°C) thereof. The extraction time is about 2 hours to seven days, depending on the extraction temperature. Additionally, during the extraction operation, for example, sodium chloride, dilute inorganic acid (e.g., dilute hydrochloric acid) or organic acid (e.g., vitamin C or tartaric acid), may be added to the extraction solution as needed to adjust the pH value of the extraction solution.

Next, the extract containing the proanthocyanidin polymer active ingredient is concentrated and dried, or partial purification or complete purification may be performed on the extract as needed. In one embodiment, for the method of the partial purification, the dried extract is re-dissolved in 95% ethanol and/or methanol aqueous solution. Next, the resulting solution is extracted using solvents with different polarities to remove partial impurities, for example, using a non-polar solvent (e.g., n-hexane) to remove lipid and non-polar substances, and then using trichloromethane and/or ethyl acetate to remove small molecule phenolic compounds. Next, the solvent-extracted liquid phase is concentrated and dried to obtain partially purified proanthocyanidin.

The complete purification method may comprise the following steps. The partially purified extract is dissolved in ethanol or methanol aqueous solution and placed into a molecular sieve column. Next, an elution is performed using different solutions and/or mixing solutions to purify and separate the proanthocyanidin. In one embodiment, the elution sequence of different solutions is 95% ethanol, 95% ethanol/methanol (1:1, v/v), 50% methanol and 50% acetone aqueous solution. The solutions which are eluted out through each eluent are collected in batches. Next, the purified proanthocyanidin in the eluted-out solution is detected using liquid chromatography (254 nm). The proanthocyanidin polymer solutions with various molecular weight distributions may be obtained by collecting the solutions which are eluted out through different eluents. Next, each of the eluted-out solution is concentrated below 40°C and freeze-dried to obtain the purified proanthocyanidin polymer. In one embodiment, the molecular sieve column used in the elution is Sephadex LH-20 column (purchased from German Amersham Corporation).

In the disclosure, the monomer units of the purified proanthocyanidin have the following formula:

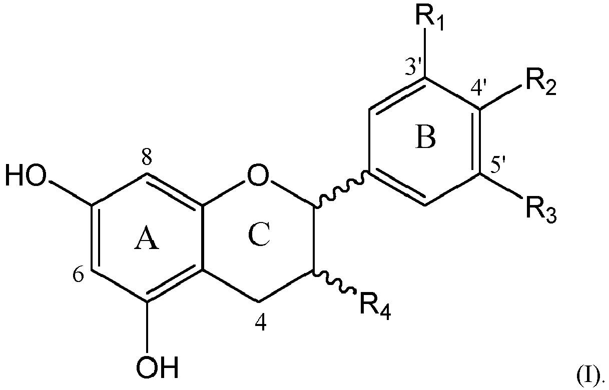

In one embodiment, when R₁ is OCH₃, R₂ is OH and R₃ is H. In another embodiment, when R₁ is OH, R₂ is H and R₃ is H. In another embodiment, when R₁ is OH, R₂ is OH and R₃ is OH. In the formula, R₄ may be 3-(α)-OH, 3-(β)-OH, 3-(α)-O-sugar or 3-(β)-O-sugar, wherein the monomer units of the proanthocyanidin are bonded to each other via C4-C8 bonding, C4-C6 bonding, or C2-C7 bonding.

The monomer units of the proanthocyanidin comprise R or S optical isomers at C2, C3 or C4.

The monomer units of proanthocyanidins can also include flavonoids such as catechin, epicatechin, epiafzetechin, gallocatechin, galloepicatechin, epigallocatechin, gallates, flavonols, flavandiols, leucocyanidins or procynidins. In one embodiment, the monomer units of proanthocyanidins comprise flavan-3-ol or flavane derivative.

The proanthocyanidin of the pharmaceutical composition disclosed in the present invention has a degree of polymerization ranging from 50 to 65; the average molecular weight is 15,000 to 19,500 Daltons, preferably 16,500 to 18,000 Daltons.

The molecular weight is determined by size exclusion chromatography using a MALS detector (SEC-MALS). MALS detectors (multi-angle light scattering detectors, such as those manufactured by Malvern Instruments Ltd. (Malvern, UK)) determine absolute molecular weights, rather than relative molecular weights (i.e., relative to a standard).

The purified proanthocyanidin in the present invention may comprise proanthocyanidin with a single degree of polymerization. It also may comprise a proanthocyanidin mixture with various degrees of polymerization.

The pharmaceutically acceptable carrier may comprise, but is not limited to, a solvent, dispersion medium, coating, antibacterial agent, antifungal agent, isotonic absorption delaying agent or pharmaceutical compatibilizer. For different modes of administration, the pharmaceutical composition may be prepared into various suitable dosage forms using known methods.

The pharmaceutically acceptable salt may comprise, but is not limited to, inorganic salts or organic salts. The inorganic salts may comprise, for example, alkali metal salts such as sodium salts, potassium salts or amine salts, alkaline earth metal salts such as magnesium salts or calcium salts, or divalent or tetravalent cation salts such as zinc salts, aluminum salts or zirconium salts. The organic salts may comprise dicyclohexylamine salts, methyl-d-glucamine salts or amino acid salts such as arginine salts, lysine salts, histidine salts or glutamine salts.

The modes of administration of the pharmaceutical composition may comprise oral, non-oral, through-inhalation-spray or through-implanted-reservoir administration. The non-oral modes may comprise subcutaneous, intracutaneous, intravenous, intramuscular, intraarticular, intraarterial, intrasynovial, intrasternal, intrathecal or intraleaional injection or perfusion techniques.

The oral dosage forms may comprise, but are not limited to, tablets, capsules, emulsions, aqueous suspensions, dispersions or solutions.

### Embodiments:

### Embodiment 1. Preparation of PPC

Roots and stems connecting from the *Boehmeria nivea L. Gaud.* medicinal material were washed and dried under a natural environment. The dried medicinal material was cut into slices having a thickness of about 5 mm and stored at 4°C. The stored Boehmeria nivea L. Gaud. medicinal material was ground by a muller and passed through a 20-mesh sieve. The obtained powder was dispersed in 95% ethanol (1:10 w/w) and thermal-refluxed for 2 hours (twice), then cooled to room temperature. The extracting solution obtained by heating and cooling to room temperature was put into a centrifuge bag to be filtered by centrifugation. The filtrate was then concentrated by a vacuum evaporator at a temperature below 40°C and then freeze-dried by a freeze dryer to obtain a proanthocyanidin-containing mixture.

The proanthocyanidin-containing mixture was dissolved in a water/ethanol solution, the ethanol was removed by a vacuum evaporator at a temperature below 40°C, and the lipid in extract was removed by adding hexane (1:10 v/v) and shaking for 30 minutes (several extractions were performed). The obtained aqueous phase was added to ethyl acetate (aqueous layer: ethyl acetate=1:1, v/v) and shaken for 30 minutes (multiple extractions were performed in this step). The resulting aqueous phase was added with 1-butanol (1:1, v/v) and shaken for 30 minutes (multiple extractions were performed in this step). The aqueous phase solution is concentrated by a vacuum evaporator at a temperature lower than 40°C, and then freeze-dried by a freeze dryer to obtain a purified proanthocyanidin-containing *Boehmeria nivea L. Gaud* extract.

The partially purified proanthocyanidin-containing *Boehmeria nivea L. Gaud* extract was separated by gel permeation chromatography (Sephadex LH-20, 45 cm long with a diameter of 4 cm) and eluted using solvents with different polar ratios to remove impurities. 2.5 g of partially purified *Boehmeria nivea L. Gaud* was dissolved in 0.5 ml of 95% ethanol and applied to a gel permeation chromatography column and eluted with a series of solvents. The eluates eluted with different solvents were collected. The solvents were 300 ml of 95% ethanol, 300 ml of 95% ethanol/methanol (1/1, v/v), 300 ml of methanol, 300 ml of 50% aqueous methanol, 300 ml of 50% aqueous acetone, and 300 ml of acetone. Except for the eluate which was eluted with 300 ml of 95% ethanol, all other eluates were concentrated by a vacuum evaporator at a temperature below 40°C and lyophilized by a freeze dryer. The lyophilized material was stored at -20°C for later use. The physicochemical properties of the lyophilized *Boehmeria nivea L. Gaud* extract with partially purified and/or purified proanthocyanidins were analyzed. The lyophilized eluate has partially purified and/or purified proanthocyanidin components.

### Embodiment 2. Structure determination of the monomer units of the proanthocyanidin polymer

The monomer unit structure of the proanthocyanidin was detected by pyrolysis gas chromatography-mass spectrometry (PGC/MS). In the detection method, solid purified proanthocyanidin (the purified sample in Embodiment 1) was directly placed into pyrolysis gas chromatography and gradually warmed or instantaneously warmed with a segmentedtemperature (50°C to 500°C) or single-temperature operation mode. The thermal-decomposed sample was separated through a specific metal column of the pyrolysis gas chromatography. The monomer unit structure of the proanthocyanidin polymer was determined by the spectrums produced from the detector of the mass spectrometry. The formula of the determined monomer unit structure of the proanthocyanidin polymer is represented as follows: 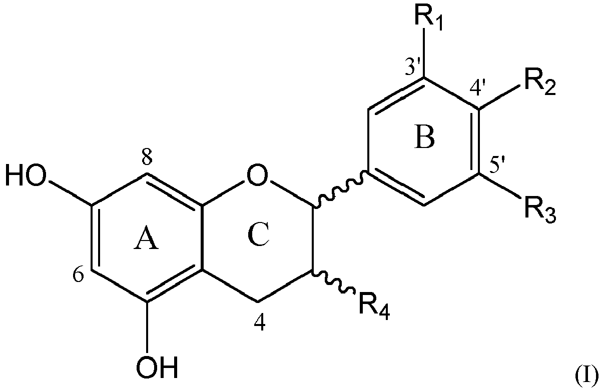 wherein, when R₁ is OCH₃, R₂ is OH and R₃ is H, or when R₁ is OH, R₂ is H and R₃ is H, or when R₁ is OH, R₂ is OH and R₃ is OH. Since the mass spectrum measured by pyrolysis gas chromatography showed a peak containing the glycoside signal, R₄ was presumed to be 3-(α)-OH, 3-(β)-OH, 3-(α)-O-sugar or 3-(β)-O-sugar; and a pharmaceutically acceptable carrier or salt.

### Embodiment 3. Molecular weight determination of proanthocyanidin polymers

### Step 1: Determination of Average Molecular Weight by SEC-MALS

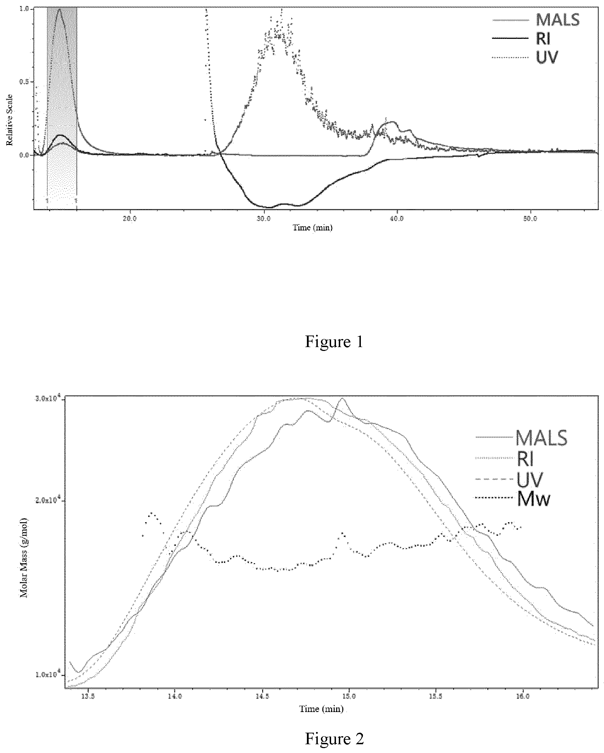

Three groups were selected from the samples obtained in embodiment 1, namely sample 1, sample 2 or sample 3. After each sample was equally divided into three parts, the mass distribution of proanthocyanidin polymer was evaluated and obtained by size exclusion chromatography using multi angle laser scattering spectrometer (SEC-MALS), and the average molecular weight was calculated. In the SEC-MALS method adopted in the present invention, a solution of ethanol + distilled water can be used as the mobile phase; the injection volume of the sample was 100 µL, the flow rate was 0.5 mL/min, and the experimental temperature was 25°C. The average molecular weight Mw of proanthocyanidin was directly obtained using the SEC-MALS method. The results of sample 1 were shown in Figures 1 and 2, and the measured average molecular weight of the sample was 16.484 kDa.

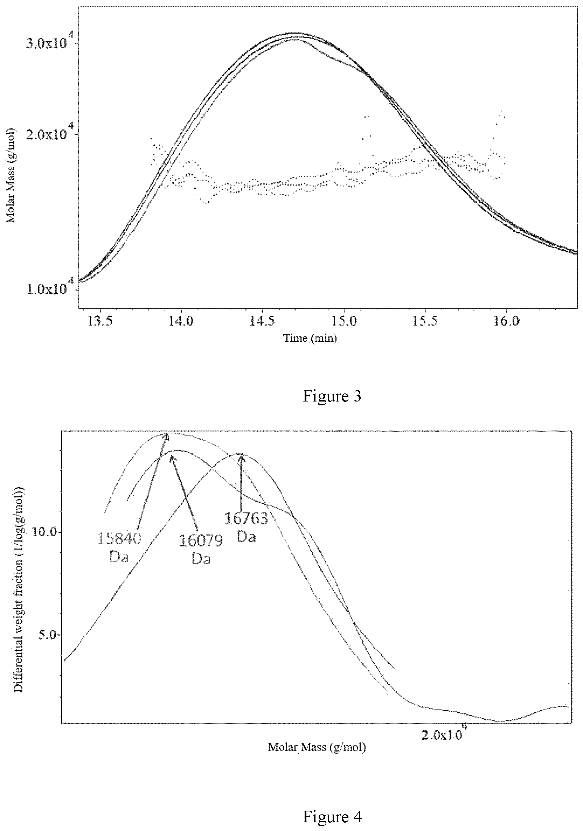

Three groups of parallel experiments were performed for each sample, and the average molecular weight of proanthocyanidin was obtained by SEC-MALS method. The repeatability of the three parallel experiments was very good. The three results of sample 1 were shown in Figure 3.

### Step 2: Polydispersity Analysis

Three groups of parallel experiments were performed for each sample in step 1, and the polydispersity was calculated according to the analysis of the asymmetric flow field flow fractionation (AF4), where the data in Table 1 was from sample 1, specifically including samples 1A, 1B and 1C.

**Table 1**

| Sample | Mn (kDa) | MW (kDa) | Polydispersity (Mw/Mn) |
|---|---|---|---|
| 1A | 16.435±2.653 | 16.484±2.666 | 1.003±0.229 |
| 1B | 16.650±2.922 | 16.703±2.899 | 1.003±0.248 |
| 1C | 16.709±2.627 | 16.799±2.579 | 1.005±0.221 |
| Average | 16.598 | 16.662 | 1.004 |
| Standard deviation | 0.202 | 0.221 | 0.001 |
| % Standard deviation | 1.214 | 1.328 | 0.136 |
| Minimum | 16.435 | 16.484 | 1.003 |
| Maximum | 16.709 | 16.799 | 1.005 |

From Table 1, it is easy to know that the weight average molecular weight (Mw) of sample 1 is 16.662 kDa, the number average molecular weight (Mn) is 16.598 kDa, the polydispersity index of the three samples is 1.004, and the concentration is very high, that is, the samples are very uniform.

### Step 3: Gel permeation chromatography (GPC) analysis of the degree of polymerization of proanthocyanidins

Three groups of parallel experiments were performed for each sample in step 1, and the corresponding spectra were obtained using AF4. The three results of sample 1 were shown in Figure 4, and the peaks of the three results were 15.840 kDa, 16.079 kDa, and 16.763 kDa, respectively.

The data of samples 1, 2 and 3 were analyzed respectively. The average molecular weights of sample 1 were 16.484kDa, 16.703kDa and 16.799kDa, respectively; the average molecular weights of sample 2 were 15.580 kDa, 15.623 kDa and 15.383 kDa, respectively. The measured average molecular weights of sample 3 were 16.325 kDa, 16.132 kDa and 16.277 kDa, respectively. Details were shown in Tables 2-1 to 2-3.

**Table 2-1**

| Polymeri zation degree | Molar mass (Da) | 1A (Mw=16.484 kDa) weight fraction (%) | 1B (Mw=16.703k Da) weight fraction (%) | 1C (Mw=16.799 kDa) weight fraction (%) |
|---|---|---|---|---|
| 50 | 15,000 | 0 | 3.7 | 0 |
| 55 | 16,500 | 51.62 | 36.85 | 62.3 |
| 60 | 18,000 | 41.16 | 50.99 | 25.44 |
| 65 | 19,500 | 7.22 | 8.46 | 7.36 |
| 70 | 21,000 | 0 | 0 | 2.15 |
| 75 | 22,500 | 0 | 0 | 2.76 |

**Table 2-2**

| Polymeri zation degree | Molar mass (Da) | 2A(Mw=15.580 kDa) weight fraction (%) | 2B (Mw=15.623 kDa) weight fraction (%) | 2C (Mw=15.383 kDa) weight fraction (%) |
|---|---|---|---|---|
| 45 | 13,500 | 1.14 | 0.46 | 1.65 |
| 50 | 15,000 | 36.52 | 29.3 | 14.02 |
| 55 | 16,500 | 44.57 | 53.59 | 83.66 |
| 60 | 18,000 | 14.89 | 16.65 | 0.67 |
| 65 | 19,500 | 2.88 | 0 | 0 |

**Table 2-3**

| Polymeri zation degree | Molar mass (Da) | 3A(Mw=16.325 kDa) weight fraction (%) | 3B (Mw=16.132 kDa) weight fraction (%) | 3C (Mw=16.277 kDa) weight fraction (%) |
|---|---|---|---|---|
| 45 | 13,500 | 0 | 0 | 1.36 |
| 50 | 15,000 | 7.52 | 5.31 | 7.97 |
| 55 | 16,500 | 51.53 | 73.68 | 58.88 |
| 60 | 18,000 | 32.66 | 16.81 | 25.62 |
| 65 | 19,500 | 8.29 | 2.24 | 3.54 |
| 70 | 21,000 | 0 | 1.02 | 2.3 |
| 75 | 22,500 | 0 | 0.94 | 0.33 |

From Tables 2-1 to 2-3, it can be seen that the polymerization degree of sample 1 is in the range of 55-65, and the molar mass distribution is 16.5 kDa~19.5 kDa; the polymerization degree of sample 2 is in the range of 50-60, and the molar mass distribution is 15.0 kDa~18.0 kDa; the polymerization degree of sample 3 is in the range of 50-65, and the molar mass distribution is in the range of 15.0 kDa to 19.5 kDa. That is, the polymerization degree of the PPC obtained by the extraction and purification of the present invention ranges from 50-65, preferably 55-60.

### Embodiment 4. Increment detection of specific refractive index

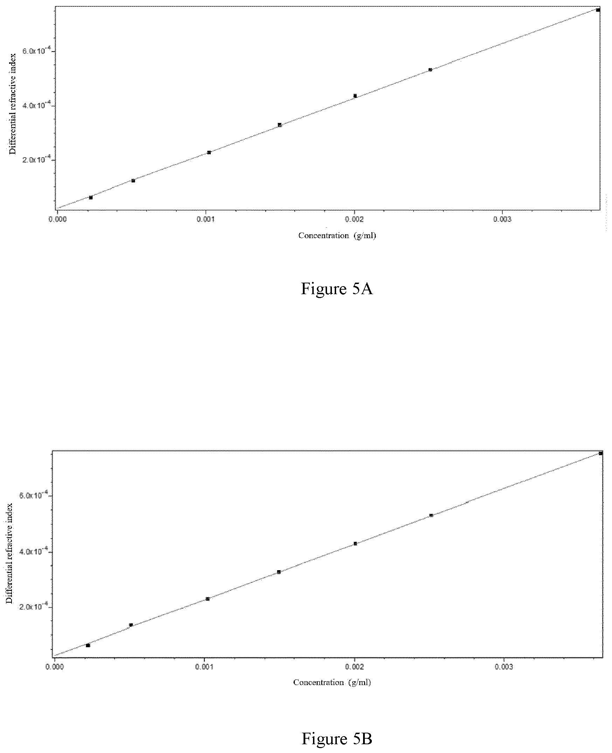

Using a GPC device equipped with a differential refractometer (RI: Optilab T-rEX), dissolved in ethanol: distilled water = 1:1 (volume), the sample was injected into the flow cell of the differential refractometer to detect the refractive index. The range of the concentration was 0.2-3.5 mg/ml, the sample flow rate was 0.3 ml/min, the experimental temperature was 25°C, the change rate of the refractive index is differentiated and measured according to the change rate of the dilute solution concentration. The results of two groups of parallel experiments were shown in Figures 5A and 5B. It can be seen that the specific refractive index increments (dn/dc) were 0.2021 ± 0.0019 mL/g and 0.1999 ± 0.0015 mL/g, respectively. The relative refractive indices (dn/dc) of the solvents for different samples were similar, indicating similar properties of the tested samples.

### Embodiment 5. Experiment of inhibition of ROS production

### Method one:

From the sample 3 in Embodiment 1, the cell lines were stimulated with MnCl₂ to induce the intracellular production of reactive oxygen species (ROS), and the separated samples of different molecular weights at different concentrations were added, to obtain data on inhibition of ROS production (revealed oxidative stress inhibition) shown in Table 3 below.

**Table 3**

| Parallel repeat experiment | Concentration (µg/ml) | Inhibition rate (%) |
|---|---|---|
| 1 | 3.125 | 16-34% |
| | 25 | 95-99% |
| 2 | 3.125 | 31% |
| | 25 | 99% |

The inhibition rate in Table 3 represents the inhibition of oxidative stress. According to the data, it can be seen that the PPC has a good ability to inhibit the production of ROS. Specifically, it has a certain inhibitory effect at low concentrations, at a higher concentration, i.e., 25 µg/ml, the inhibition rate almost reached 99%.

### Method two:

Cell culture: Human hepatoma cells (Huh7) were cultured in MEM. The medium was supplemented with 1% Penicillin/Streptomycin mixture and 1% non-essential amino acids, 1% GlutaMAX-I, 1 mM sodium pyruvate and 10% fetal bovine serum. Cells were cultured in a 37°C, 5% CO₂ incubator.

10 mM general oxidative stress indicator CM-H₂DCFDA was added to the cells, incubated at 37°C for 15 minutes, and after co-incubation with fluorescent probes, the cells were washed twice with PBS. The cells were induced to produce ROS with H₂O₂, and the samples were tested for their inhibitory activity against ROS. The PPC of the present invention is hereinafter referred to as BEL-X.

Experimental group settings:
(1) Blank control: cells without any treatment;
(2) H₂O₂ treatment: 0.5 mM or 1 mM H₂O₂ medium was added to induce cells to produce ROS;
(3) BEL-X and H₂O₂ treatment: 5 µg/ml or 10 µg/ml BEL-X was added to the culture medium with 0.5 mM or 1 mM H₂O₂, respectively.

The cells in the above groups were cultured for 2 hours, and the fluorescence intensities were measured with a spectrophotometer to calculate the inhibitory effect of BEL-X on H₂O₂induced ROS production in cells. The data obtained were shown in Table 4.

**Table 4**

| Concentration of H₂O₂ (mM) | Concentration (µg/ml) | Inhibition rate (%) |
|---|---|---|
| 0.5 | 5 | 88.6 |
| | 10 | 100 |
| 1.0 | 5 | 82.4 |
| | 10 | 96.2 |

The inhibition rates in Table 4 were characterized by the inhibitory effect of H₂O₂-induced ROS production in cells in the test sample. According to the data, it can be seen that the purified PPC has a good ability to inhibit the production of ROS.

### Method three:

Cell culture: Human hepatoma cells (Huh7) were cultured in MEM, and human colorectal cancer cells (HRT-18) were cultured in DMEM. The above media were supplemented with 1% Penicillin/Streptomycin mixture and 1% non-essential amino acids, 1% GlutaMAX-I, 1 mM sodium pyruvate and 10% fetal bovine serum. Cells were cultured in a 37°C, 5% CO₂ incubator.

The cells were induced to produce ROS with MnCl₃, and the inhibitory activity of the samples against ROS was tested.

Experimental group settings:
(1) Blank control: cells without any treatment;
(2) DMSO treatment: DMSO was added to the solution, the DMSO concentration was 1.4 mg/ml;
(3) BEL-X treatment: The sample BEL-X of the present invention was dissolved in a solvent, and the final concentration was 25 µg/ml;
(4) MnCl₂ induction: 200 µM MnCl₂ was added;
(5) Co-treatment of BEL-X and MnCl₂: 200 µM MnCl₂ and 25 µg/ml BEL-X samples were added; the above five groups of cells were each cultured in a 37°C, 5% CO₂ incubator for 4 hours;
(6) BEL-X-pre-treatment + MnCl₂: After adding 25 µg/ml BEL-X sample, the cells were cultured for 4 hours, and then 200 µM MnCl₂ was added and then cultured for 2 hours.

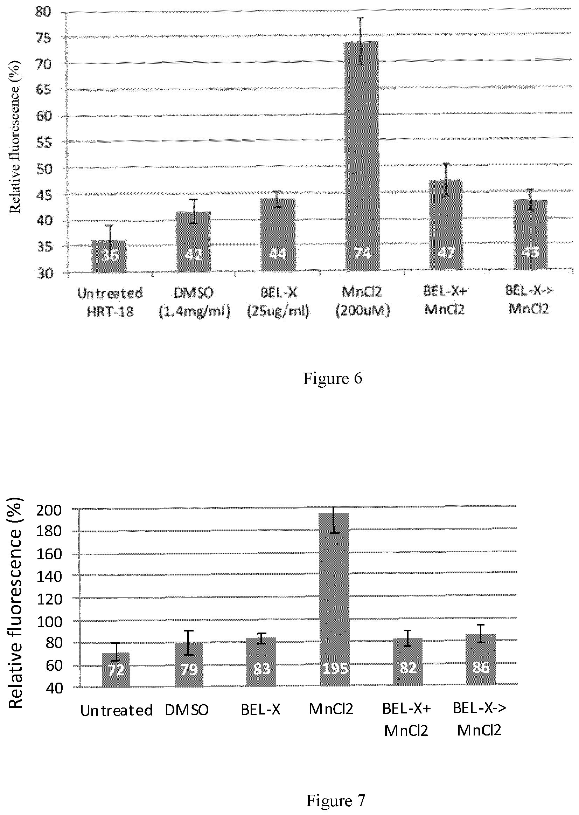

Detection of ROS production: 10 mM CM-H₂DCFDA was added to the above cells and incubated at 37°C for 45 minutes. After incubation with fluorescent probes, cells were washed twice with PBS, and the fluorescence intensities were measured with a spectrophotometer to obtain ROS data as shown in Figures 6-7.

It can be seen from Figures 6-7 that PPC can inhibit the production of ROS induced by MnCl₂.

### Embodiment 6. Comparison experiments on inhibition of active oxides at different degrees of polymerization

Cell culture: Human colorectal cancer cells (HRT-18) were cultured in MEM. The medium was supplemented with 1% Penicillin/Streptomycin mixture and 1% non-essential amino acids, 1% GlutaMAX-I, 1 mM sodium pyruvate and 10% fetal bovine serum. Cells were cultured in a 37°C, 5% CO₂ incubator.

The cells were induced to produce ROS with MnCl₃, and the inhibitory activity of the samples against ROS was tested.

Experimental group settings:
(1) Blank control: cells without any treatment;
(2) DMSO treatment: DMSO was added until the DMSO concentration in the solution was 1.4 mg/ml;
(3) MnCl₂ treatment: 200 µM MnCl₂ was added to induce cells to produce ROS;
(4) BEL-X treatment: The sample BEL-X of the present invention was dissolved in a solvent, and the final concentration was 25 µg/ml;
(5) Co-treatment of BEL-X and MnCl₂: 200 µM MnCl₂ and BEL-X (25 µg/ml) were added to the sample;
(6) BEL-X-molecular weight >3K treatment: The sample BEL-X (molecular weight greater than 3 kDa) was dissolved in the solvent, then diluted and added to the cell culture medium, the final concentration was 25 µg/ml;
(7) Co-treatment of BEL-X-molecular weight >3K and MnCl₂: 200 µM MnCl₂ and 25 µg/ml BEL-X (molecular weight >3kDa) were added to the cell culture medium at the same time;
(8) BEL-X-molecular weight <3K treatment: The sample BEL-X (molecular weight less than 3 kDa) was dissolved in the solvent, then diluted and added to the cell culture medium, the final concentration was 25 µg/ml;
(9) Co-treatment of BEL-X-molecular weight <3K and MnCl₂: 200 µM MnCl₂ and BEL-X (molecular weight less than 3 kDa) at a concentration of 25 µg/ml were added to the cell culture medium at the same time.

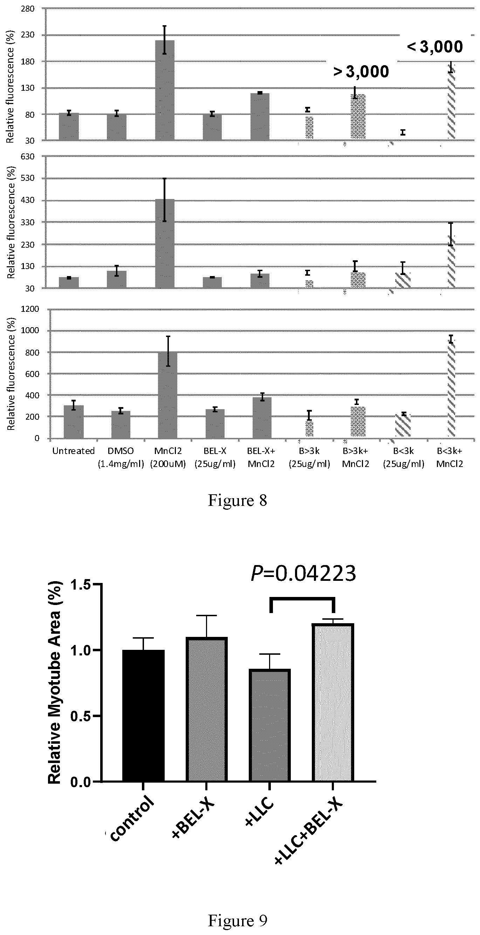

Detection of ROS production: 10 mM CM-H₂DCFDA was added to the above cells and incubated at 37°C for 45 minutes. After incubation with fluorescent probes, cells were washed twice with PBS, and the fluorescence intensities were measured with a spectrophotometer to obtain ROS data as shown in Figure 8.

It can be seen from Figure 8 that the PPC can effectively inhibit the ROS induced by MnCl₃, while the oligomeric proanthocyanidins have no ability to inhibit the ROS generation.

### Embodiment 7. Experiment in PPC improving cancer-induced sarcopenia

### Method one:

Cell culture: Mouse myoblasts (C2C12) were cultured in DMEM medium containing 10% FBS at 37°C and CO₂ incubator for 24-72 hours.

Horse serum induction: The above cells were co-cultured with DMEM medium containing 2% horse serum for 72 hours to induce myotube differentiation.

Experimental group settings:
(1) Blank control: Cells were cultured in DMEM medium for 24 hours after induction;
(2) BEL-X treatment: The sample BEL-X of the present invention was dissolved in a solvent, then added to the cell culture medium, the final concentration of BEL-X was 50 µg/ml, and then cultured with cells for 24 hours;
(3) LLC treatment: lung cancer conditioned medium (LLC) was added to the medium so that the LLC concentration was 25%, and cultured with the cells for 24 hours;
(4) LLC + BEL-X co-treatment: The sample BEL-X of the present invention was dissolved in a solvent, then added to a cell culture medium containing 25% LLC, the final concentration of BEL-X was 50 µg/m, and then cultured for 24 Hours.

Detection of mysoin heavy chain (MyHC): The above 4 groups of cells were collected, C2C12 cells were allowed to grow and form myotubes, after rinsing with phosphate-buffered saline (PBS), the cells were fixed in 100% methanol pre-cooled at -20°C for 5 minutes. Then, the cells were naturally dried and stored at 4°C. The fixed cells were re-dissolved in PBS containing 0.5% tween 20 and placed on ice for 10 minutes, the cell membranes were perforated, and then treated with PBS containing 0.1% tween 20 and 0.2% BSA for 1 hour, and then mixed with MHC primary antibody (Millipore) at room temperature for 1 hour. Then, the cells were washed twice with PBS, reacted with mouse anti-MHC secondary antibody (Invitrogen) for 1 hour, and the nuclei were stained with DAPI (4',6-diamidino-2-phenylindole) stain. Immunostained myotubes were observed with a fluorescence microscope, and images were captured using Image J software (NIH, Bethesda, MD, USA). The area of at least 100 myotubes was measured for each experimental group, and the average area of each myotube was calculated as Average of three measurements. The obtained experimental results are plotted as shown in Figure 9.

It can be seen from Figure 9 that lung cancer conditioned medium (LLC) can induce the atrophy of C2C12 myotube cells. Therefore, when C2C12 myotube cells were co-cultured with LLC, the expression of myosin heavy chain was significantly decreased, while the expression of myosin heavy chain was restored after adding BEL-X, which proves that the BEL-X of the present invention can effectively inhibit the atrophy of the C2C12 myotube cells, and further contributes to the treatment and prevention of sarcopenia.

### Method Two:

The cell culture and experimental group settings were the same as in above Method one.

LLC induction: The cells were transferred into lung cancer conditioned medium (LLC) to induce Atrogin-1 and MuRF-1 gene expression, i.e., a phenomenon of muscle atrophy.

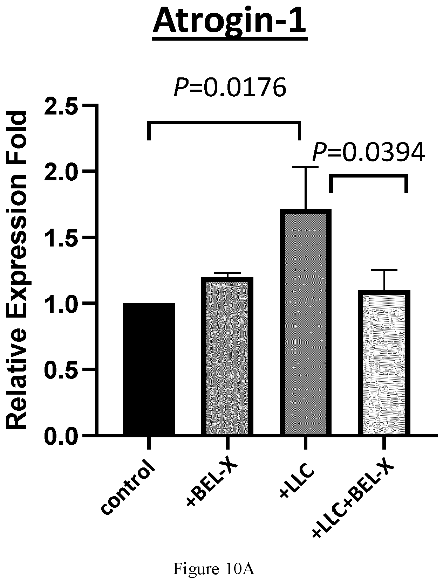

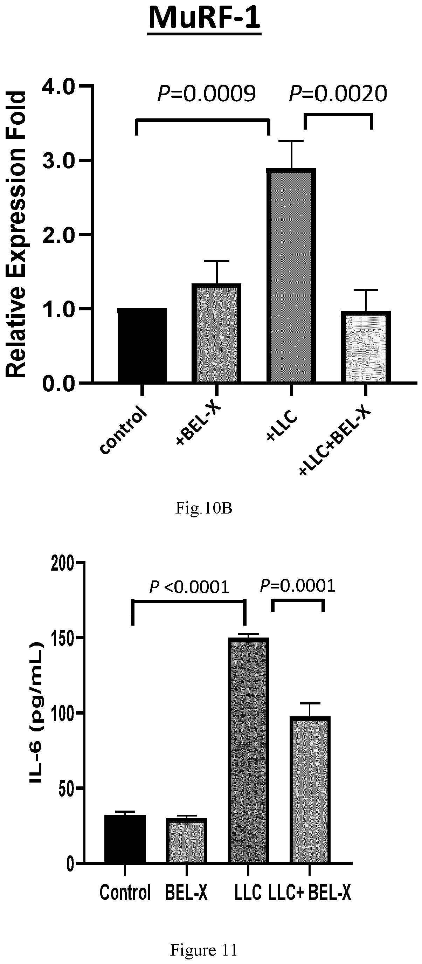

RT-PCR detection of gene expression in C2C12 myotube cells: RNAs were extracted using Qiagen RNeasy kit, and single-stranded cDNAs were synthesized using Biora iScript kit. RT-PCR was performed using Riorad iQ SYBR green supermix. The amplification conditions were (1) 95°C for 30 min, (2) 95°C for 15 sec, and 60°C for 45 sec, 40 cycles. The amount of target mRNAs was normalized against the Actin gene. The obtained experimental results were plotted as shown in Figures 10A and 10B.

It can be seen from Figures 10A and 10B that LLC can induce the expression of Atrogin-1 and MuRF-1 genes in C2C12 myotube cells, thereby causing cell atrophy. Therefore, when C2C12 myotube cells were co-cultured with LLC, the gene expression was significantly increased, while gene expression was restored after adding BEL-X, that is, the BEL-X of the present invention can effectively prevent the expression of Atrogin-1 and MuRF-1 genes in C2C12 myotube cells, which is further helpful for the treatment and prevention of sarcopenia.

### Method three:

The cell culture, myotube formation in C2C12 cells induced by horse serum, and experimental group settings were the same as in Method one.

ELISA detection of interleukin-6 (IL-6): Mouse IL-6 Quantikine ELISA kit (R&D systems) was used to detect the content of interleukin-6 according to its detection steps. The obtained experimental results were plotted as shown in Figure 11.

It can be seen from Figure 11 that the addition of LLC to C2C12 myotube cells produced a large amount of IL-6, that is, LLC can induce the inflammatory response of C2C12 myotube cells; in addition, after adding BEL-X, the expression of IL-6 was significantly decreased, that is, the BEL-X of the present invention has an anti-inflammatory effect.

### Embodiment 8. PPC improves sarcopenia caused by oxidative stress

### Method one:

The cell culture and myotube formation in C2C12 cells induced by horse serum were the same as in Method one of Embodiment 7.

Experimental group settings:
(1) Blank control: Cells were cultured in medium for 24 hours after induction;
(2) BEL-X treatment: The sample BEL-X of the present invention was dissolved in a solvent, then added to the cell culture medium so that the concentration of BEL-X was 50 µg/ml, and cultured with the cells for 24 hours;
(3) H₂O₂ treatment: H₂O₂ was added to the medium so that the concentration of H₂O₂ was 100 µM, and cultured with cells for 24 hours;
(4) Co-treatment of H₂O₂ + BEL-X: The sample BEL-X of the present invention was dissolved in a solvent, and then added to a cell culture medium containing H₂O₂, so that the concentration of BEL-X was 50 µg/ml, and then cultured for 24 hours.

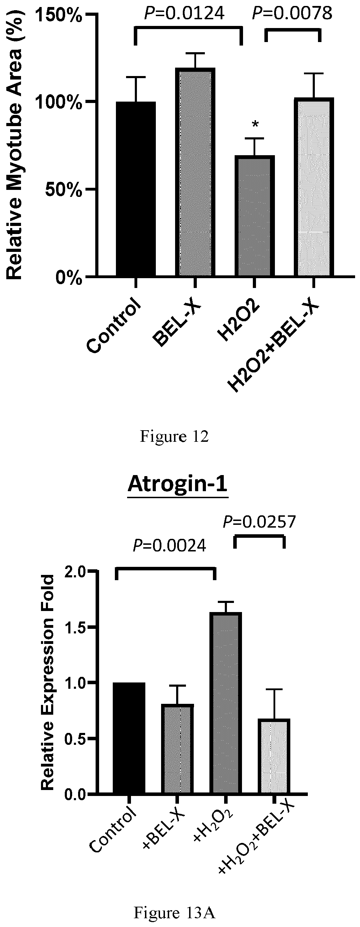

The method for detecting myosin heavy chain was consistent with that described in method one in Embodiment 7, and the obtained results were plotted as shown in Figure 12.

It can be seen from Figure 12 that, first of all, BEL-X of the present invention can enhance the formation of myotubes, while H₂O₂ can reduce the formation of myotubes in C2C12 cells. Therefore, after adding H₂O₂ to the culture medium of C2C12 myotube cells, the expression of myosin heavy chain was significantly reduced. The expression of myosin heavy chain was restored after adding BEL-X, that is, the BEL-X of the present invention can enhance the formation of myotubes, and can also effectively prevent H₂O₂ from damaging myotubes, which is further helpful for treatment and prevention of sarcopenia.

### Method Two:

The methods of cell culture and RT-PCR detection of gene expression in C2C12 myotube cells were the same as those of method two in Embodiment 7.

ROS induction: The cells were transferred to medium containing H₂O₂, thereby inducing Atrogin-1 or MuRF-1 gene expression, a phenomenon of muscle damage.

The experimental group settings were the same as those in Method one of Embodiment 8.

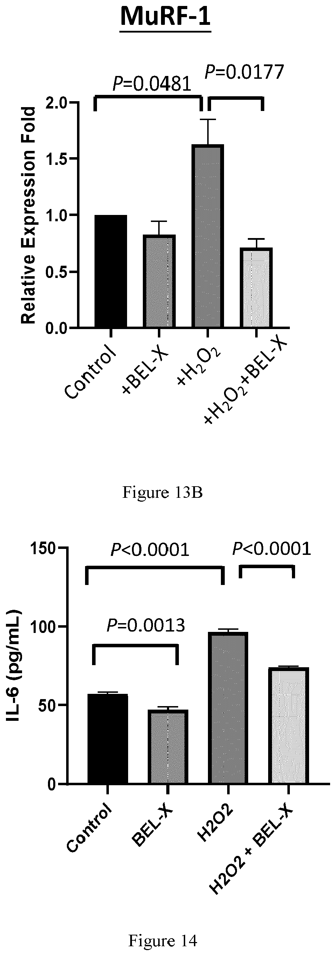

The resulting plots were shown in Figures 13A and 13B. It can be seen from Figures 13A and 13B that H₂O₂ can induce the expression of Atrogin-1 and MuRF-1 genes in C2C12 myotube cells, thereby causing myotube damage. Therefore, when C2C12 myotube cells were co-cultured with H₂O₂, the gene expression was significantly increased, while the gene expression level was restored or even lower than that of the control group after adding BEL-X. That is, the BEL-X of the present invention can effectively prevent the expression of Atrogin-1 and MuRF-1 genes in C2C12 myotube cells, which is further helpful for the treatment and prevention of sarcopenia.

### Method three:

The methods of cell culture and detection of interleukin-6 were the same as those of method three in Embodiment 7.

ROS induction and experimental group settings were the same as those in Method two in Embodiment 8.

The resulting plot was shown in Figure 14. It can be seen from Figure 14 that H₂O₂ can induce a large amount of IL-6 in C2C12 myotube cells, which can induce an inflammatory response in the cells; and after adding BEL-X, the expression of IL-6 was significantly decreased, that is, BEL-X of the present invention can reduce H₂O₂-induced IL-6 production, and has anti-inflammatory effects.

## Claims

1. A pharmaceutical composition comprising an effective amount of proanthocyanidin, wherein the monomer units of the proanthocyanidin have the following formula: 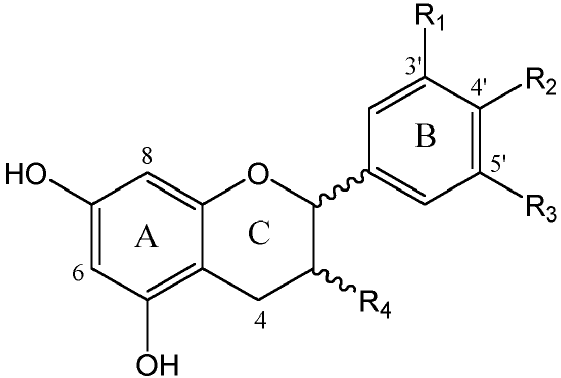
wherein, when R₁ is OCH₃, R₂ is OH and R₃ is H, when R₁ is OH, R₂ is H and R₃ is H, when R₁ is OH, R₂ is OH and R₃ is H, or when R₁ is OH, R₂ is OH and R₃ is OH; and R₄ is 3-(α)-OH, 3-(β)-OH, 3-(α)-O-sugar or 3-(β)-O-sugar; wherein the proanthocyanidin has a degree of polymerization ranging from 50 to 65; and
a pharmaceutically acceptable carrier or salt.

2. The pharmaceutical composition according to claim 1, wherein the monomer units of the proanthocyanidin are bonded to each other via C4-C8 bonding, C4-C6 bonding, or C2-C7 bonding.

3. The pharmaceutical composition according to claim 1, wherein the monomer units of the proanthocyanidin comprise R or S optical isomers at C2, C3 or C4.

4. The pharmaceutical composition according to claim 1, wherein the flavonoid compounds comprise gallocatechin, galloepicatechin or epigallocatechin.

5. The pharmaceutical composition according to claim 1, wherein the pharmaceutical composition comprises catechin, epicatechin, epiafzetechin, gallates, flavonols, flavandiols, leucocyanidins or procynidins.

6. The pharmaceutical composition according to claim 1, wherein the pharmaceutical composition comprises flavan-3-ol.

7. The pharmaceutical composition according to claim 1, wherein the proanthocyanidin has a degree of polymerization ranging from 55 to 60.

8. The pharmaceutical composition according to claim 1, wherein the pharmaceutically acceptable salt includes sodium salt, potassium salt, amine salt, magnesium salt, calcium salt, zinc salt, aluminum salt, zirconium salt, dicyclohexylamine salt, methyl-D-glucosamine salt, arginine salt, lysine salt, histidine salt or glutamine salt.

9. The use of pharmaceutical composition according to claim 1, to treat or prevent brain diseases, aging related diseases caused by the generation of active oxides, or to prevent and treat aging.

10. The use of pharmaceutical composition according to claim 9, for treating and preventing diseases such as liver disease, tumor and sarcopenia.
